# FASCICULE DE BREVET EUROPEEN

(11) **EP 2 683 649 B1**
(45) Date de publication et mention de la délivrance du brevet: **07.06.2017**
(21) Numéro de dépôt: 12706870.8
(22) Date de dépôt: 06.03.2012
(51) Int. Cl.: B82Y 30/00, C07K 14/00, C07K 14/47

(54) **NANOFIL ELECTROCONDUCTEUR BIODEGRADABLE, SON PROCEDE DE FABRICATION ET SES UTILISATIONS**
BIOLOGISCH ABBAUBARER ELEKTROLEITFÄHIGER NANODRAHT, SEINE HERSTELLUNG UND ANWENDUNG
BIODEGRADABLE ELECTROCONDUCTING NANOWIRE, ITS METHOD OF MANUFACTURE AND ITS USES

(30) Priorité: 07.03.2011 FR 1151851
(43) Date de publication de la demande: 15.01.2014
(73) Titulaire: Commissariat à l'Énergie Atomique et aux Énergies Alternatives, 75015 Paris (FR)
(72) Inventeur: FORGE, Vincent, F-38210 Vourey (FR); HORVATH, Christophe, F-74140 Loisin (FR); FONTECAVE, Marc, F-38330 Saint Ismier (FR); DURAFFOURG, Nicolas, 38470 Vinay (FR)
(74) Mandataire: Ahner, Philippe
(86) Numéro de dépôt international: PCT/EP2012/053824
(87) Numéro de publication internationale: WO 2012/120013

(56) Documents cités:
- WO-A1-00/39310
- WO-A2-2005/118633
- CAROLYN E. LUBNER ET AL: "Wiring Photosystem I for Direct Solar Hydrogen Production", BIOCHEMISTRY, vol. 49, no. 3, 26 janvier 2010 (2010-01-26), pages 404-414, XP055011946, ISSN: 0006-2960, DOI: 10.1021/bi901704v
- PADALKAR S ET AL: "Protein-templated semiconductor nanoparticle chains", NANOTECHNOLOGY, vol. 19, no. 27, 9 juillet 2008 (2008-07-09), page 275602, XP020136709, IOP, BRISTOL, GB ISSN: 0957-4484
- SCHEIBEL T ET AL: "CONDUCTING NANOWIRES BUILT BY CONTROLLED SELF-ASSEMBLY OF AMYLOID FIBERS AND SELECTIVE METAL DEPOSITION", PROCEEDINGS OF THE NATIONAL ACADEMY OF SCIENCES OF USA,, vol. 100, no. 8, 15 avril 2003 (2003-04-15), pages 4527-4532, XP001180754, NATIONAL ACADEMY OF SCIENCE, WASHINGTON, DC; US ISSN: 0027-8424, DOI: 10.1073/PNAS.0431081100
- L. L. DEL MERCATO ET AL: "Charge transport and intrinsic fluorescence in amyloid-like fibrils", PROCEEDINGS OF THE NATIONAL ACADEMY OF SCIENCES, vol. 104, no. 46, 1 janvier 2007 (2007-01-01), pages 18019-18024, XP055012138, ISSN: 0027-8424, DOI: 10.1073/pnas.0702843104
- EMMANOUIL KASOTAKIS ET AL: "Design of metal-binding sites onto self-assembled peptide fibrils", BIOPOLYMERS, vol. 92, no. 3, 1 janvier 2009 (2009-01-01), pages 164-172, XP055012169, ISSN: 0006-3525, DOI: 10.1002/bip.21163
- MAHIAR HAMEDI ET AL: "Electrochemical Devices Made from Conducting Nanowire Networks Self-Assembled from Amyloid Fibrils and Alkoxysulfonate PEDOT", NANO LETTERS, vol. 8, no. 6, 1 juin 2008 (2008-06-01), pages 1736-1740, XP055011979, ISSN: 1530-6984, DOI: 10.1021/nl0808233
- ANDREW J. BALDWIN ET AL: "Cytochrome Display on Amyloid Fibrils", JOURNAL OF THE AMERICAN CHEMICAL SOCIETY, vol. 128, no. 7, 1 février 2006 (2006-02-01), pages 2162-2163, XP055012199, ISSN: 0002-7863, DOI: 10.1021/ja0565673
- Taber et al.: "Development and Characterization of Conductive Amyloid Fibril Nanowires", American Chemical Society 64th Northwest Regional Meeting (NORM 2009) , no. Abst.72881 2009, XP002673563, Extrait de l'Internet: URL:http://acs.confex.com/acs/norm09/webpr ogram/Paper72881.html [extrait le 2012-04-10]

## Description

### DOMAINE TECHNIQUE

La présente invention appartient au domaine technique des nanofils électroconducteurs, de leur fabrication et de leurs utilisations.

Plus spécifiquement, elle se rapporte à des nanofils qui, outre de présenter des propriétés de conduction électrique, sont biodégradables, c'est-à-dire qu'ils peuvent être entièrement dégradés par des organismes vivants, et sont dotés de propriétés d'auto-organisation tant à l'échelle nanoscopique que macroscopique.

L'invention se rapporte également à un procédé permettant de fabriquer ces nanofils ainsi qu'aux utilisations desdits nanofils.

Compte tenu de leurs propriétés remarquables, les nanofils selon l'invention sont susceptibles de trouver de nombreuses applications à la fois nanoscopiques et macroscopiques.

Ainsi, ils peuvent notamment être utilisés dans des capteurs, et en particulier des capteurs destinés à détecter des espèces chimiques ou biologiques, dans des nanobatteries, et en particulier dans des nanobatteries destinées à transformer l'énergie solaire en électricité, dans des systèmes de radio-identification ainsi que dans toutes les applications de l'électronique moléculaire comme, par exemple, la fabrication de systèmes de mémoire moléculaire.

### ÉTAT DE LA TECHNIQUE ANTÉRIEURE

Le défi de la miniaturisation et la recherche de nouveaux matériaux aux propriétés originales ont amené la technologie à entrer dans l'échelle du nanomètre.

Ainsi, de nombreux travaux ont été consacrés ces dernières années à la conception et à la réalisation de nano-objets, c'est-à-dire d'objets dont l'une au moins des dimensions est comprise entre 1 et 100 nm.

Du fait de leur très faible taille, les nano-objets ont généralement des propriétés inédites par rapport aux objets de même composition chimique mais de taille plus importante en sorte que leurs applications sont potentiellement très nombreuses et touchent des domaines extrêmement variés comme l'électronique, la microélectronique, l'optique, la médecine, la pharmacie, la cosmétique, l'automobile, l'aéronautique, etc.

Toutefois, ces nano-objets suscitent de nombreuses interrogations en terme de santé publique.

En effet, on sait qu'ils peuvent pénétrer dans l'organisme par les voies cutanée, digestive et respiratoire, et leur présence dans les tissus cellulaires pourrait s'avérer, à terme, dangereuse et ce, d'autant plus que les nano-objets proposés à ce jour ne sont pas biodégradables ou ne le sont, dans le meilleur des cas, que très partiellement.

En ce qui concerne les nanofils électroconducteurs, qui représentent une catégorie particulière de nano-objets, il a été proposé à ce jour des nanotubes de carbone, des nanofils inorganiques, des nanofils de polymères conducteurs, des nanofils de polymères redox et des nanofils hybrides organique/ inorganique.

Les nanotubes de carbone sont constitués d'un ou plusieurs feuillets de carbone graphite enroulés sur eux-mêmes qui forment ainsi un cylindre. Ils peuvent être obtenus en créant un arc électrique sur une électrode de carbone ou par dépôt chimique en phase vapeur, plus connu sous le sigle CVD (de « **C**hemical **V**apor **D**eposition »). Abondamment étudiés depuis leur invention, les nanotubes de carbone ont des propriétés de conduction électrique qui varient en fonction de leurs caractéristiques structurelles, une excellente résistance mécanique, une bonne conductivité thermique, une grande capacité de greffage et des propriétés optiques non-linéaires. Mais les premières études de toxicité réalisées chez l'animal montrent qu'ils pourraient induire chez l'homme des lésions analogues à celles provoquées par l'amiante (Jones et Grainger, Advanced Drug Delivery Reviews 2009, 61(6), pages 438-456, **[1]**).

Les nanofils inorganiques sont constitués de métaux comme l'or, le platine ou le nickel, de métalloïdes comme le silicium ou le germanium, d'oxydes métalliques comme l'oxyde de zinc, ou d'alliages métal/métalloïde comme l'arséniure de gallium. Ils sont typiquement réalisés par croissance sur la surface d'un substrat par CVD. Il se forme ainsi un réseau de nanofils disposés perpendiculairement à la surface du substrat, qui présente l'avantage de pouvoir être directement intégré dans des dispositifs du type transistors à effet de champ, capteurs ou analogues. Ces nanofils inorganiques ne sont évidemment pas biodégradables.

Les nanofils de polymères conducteurs sont formés d'un enchaînement de monomères aromatiques du type thiophène ou analogues, le long duquel les électrons des doubles liaisons peuvent se déplacer et créer un courant électrique. Les polymères conducteurs sont issus de l'industrie des polymères et peuvent être fabriqués en grande quantité. Par contre, outre qu'ils ne sont pas biodégradables, ils sont dénués de propriétés d'auto-organisation, pourtant quasi indispensables pour une utilisation de nanofils à une échelle nanoscopique. Il existe d'ailleurs peu de données dans la littérature sur d'éventuelles applications nanoscopiques de ce type de nanofil.

Les nanofils de polymères redox sont, comme les précédents, formés d'un enchaînement de monomères aromatiques mais sur lesquels sont greffés de façon régulière des centres redox qui peuvent accepter ou donner un électron en changeant d'état d'oxydation. Ainsi, un électron peut être donné par un centre redox et accepté par un autre, ce qui lui permet de se déplacer le long du nanofil par sauts successifs de centre redox en centre redox. Mis à part le mode et la qualité du transport électronique, les nanofils de polymères redox présentent les mêmes avantages et les mêmes inconvénients que les nanofils de polymères conducteurs précédemment évoqués. En particulier, ils ne sont pas biodégradables et leurs applications sont également quasi exclusivement macroscopiques.

Quant aux nanofils hybrides organique/ inorganique, ils sont obtenus en liant, par des liaisons covalentes ou par des interactions électrostatiques, des colloïdes métalliques en plusieurs endroits de brins d'ADN ou de fibres amyloïdes et en faisant croître du métal sur ces colloïdes jusqu'à ce que ces derniers entrent en contact les uns avec les autres et soient capables d'assurer une conduction électrique à la surface desdits brins d'ADN ou desdites fibres amyloïdes. Est ainsi créée une gaine métallique autour des brins d'ADN ou des fibres amyloïdes, qui doit être suffisamment épaisse pour assurer une conduction électrique satisfaisante. Ces nanofils sont donc assimilables à des nanofils inorganiques que ce soit en termes de conductivité électrique, de solidité et de stabilité mais aussi en ce qui concerne leur incapacité à être dégradés par des organismes vivants. Un exemple de ce type de nanofil, comprenant une fibre amyloïde issue du domaine NM de la protéine prion Sup35p de *Saccharomyces cerevisiae* et recouverte d'argent et d'or, est décrite dans WO-A-2005/118 633 **[9]**.

Par ailleurs, on connait par Baldwin et al. (J. Am. Chem. Soc. 2006, 128, 2162-2163, **[10]**, une fibre amyloïde fonctionnalisée par le cytochrome b₅₆₂ *d'Escherichia coli.* Cette fibre amyloïde est obtenue à partir d'une protéine chimère comprenant la séquence peptidique du cytochrome b₅₆₂, encadrée de part et d'autres par la séquence peptidique d'un domaine SH3. Les deux domaines SH3 de chaque protéine interagissent ensemble et avec leurs homologues d'autres protéines pour former une fibre amyloïde. La séquence peptidique correspondant au cytochrome se retrouve liée de façon covalente à la fibre amyloïde après sa formation. D'abord déplié, le cytochrome retrouve sa conformation après l'ajout d'une porphyrine. La possibilité que la fibre amyloïde ainsi fonctionnalisée conduise le courant par sauts électroniques de porphyrine enporphyrine est évoquée sans être toutefois démontrée.

Compte tenu de ce qui précède, les Inventeurs se sont donc fixé pour but général de fournir des nanofils qui, tout en étant dotés de propriétés de conduction électrique, soient biodégradables.

Ils se sont également fixé pour but que ces nanofils puissent être utilisés tant à une échelle nanoscopique, microscopique que macroscopique.

Ils se sont aussi fixé pour but que ces nanofils puissent être aisément fabriqués à une échelle industrielle et à un coût tel qu'ils puissent trouver des applications dans de très nombreux domaines.

En particulier, ils se sont fixé pour but que ces nanofils puissent être fabriqués par des procédés conventionnels.

### EXPOSÉ DE L'INVENTION

Ces buts et d'autres encore sont atteints par l'invention qui propose, en premier lieu, un nanofil électroconducteur, lequel comprend une fibre amyloïde qui résulte de l'auto-assemblage de peptides comprenant un domaine prion ou un dérivé de ce domaine, ce dérivé étant capable de former des fibres amyloïdes et ayant une séquence d'acides aminés qui présente au moins 75% d'identité avec la séquence d'acides aminés du domaine prion dont il est dérivé, et dans lequel la fibre amyloïde est fonctionnalisée par des peptides transporteurs d'électrons, comprenant chacun un ou plusieurs acides aminés liés à un ou plusieurs atomes de fer, les peptides transporteurs d'électrons étant issus d'une protéine à centre fer-soufre.

Dans ce qui précède et ce qui suit, on entend par *« nanofil* », un fil dont l'épaisseur est comprise entre 1 et 100 nanomètres mais dont la longueur peut, elle, aller jusqu'à 10 micromètres.

On rappelle que les fibres amyloïdes sont des fibres qui résultent de l'auto-assemblage de protéines. Cet auto-assemblage a pour caractéristique de s'auto-propager puisque l'ajout d'une faible quantité d'une protéine sous forme de fibres amyloïdes dans une suspension de cette même protéine accélère le passage de la quasi-totalité de la protéine sous forme de fibres amyloïdes.

Les fibres amyloïdes présentent une structure caractéristique en feuillets β intermoléculaires. Elles montrent une coloration au rouge Congo, associée à une biréfringence à la lumière polarisée (Sipe et Cohen, Journal of Structural Biology 2000, 130(2-3), pages 88-98, **[2]**), et provoquent une forte augmentation de la fluorescence émise par la thioflavine-T à la longueur d'onde de 480 nm (Sabate et al., Journal of Structural Biology 2008, 162(3), pages 387-396, **[3]**). Elles possèdent également un profil de diffraction aux rayons X qui est caractéristique d'une structure cross-bêta.

Les fibres amyloïdes ont d'abord été détectées dans des dépôts protéiques associés à des maladies neurodégénératives comme les maladies d'Alzheimer et de Parkinson. Ces dépôts sont la conséquence d'un mauvais repliement, avec perte d'activité biologique, des protéines concernées. Ce phénomène est corrélé à une déstabilisation de la structure native de ces protéines par une mutation ou par une modification post-traductionnelle (protéolyse, phosphorylation, etc) due à un stress. Les fibres amyloïdes ont fait l'objet de très nombreuses études destinées à comprendre leurs mécanismes de formation dans les milieux cellulaires et leurs modes de propagation.

Dans le cadre de ces travaux, une nouvelle classe de protéines a été découverte, à savoir les protéines à domaine prion (Baxa et al., Advances in Protein Chemistry 2006, 73, pages 125-180, **[4]**). Ces protéines ont tout d'abord été mises en évidence chez les levures puis chez les champignons mais un nombre croissant de travaux suggère qu'elles seraient également présentes chez les êtres humains.

Les domaines prions forment des fibres qui présentent les principales caractéristiques structurales des fibres amyloïdes : feuillets β, fixation de marqueurs spécifiques, etc. On désigne donc également ces fibres sous le nom de fibres amyloïdes.

Cependant, dans le cas des protéines à domaine prion, la formation des fibres amyloïdes n'est pas due à un mauvais repliement de ces protéines mais fait partie intégrante de leur activité biologique et intervient dans des conditions physiologiques et, en particulier, à pH neutre. Elle permet en général la régulation de la fonction d'un autre domaine des protéines. La structuration des domaines prions dans les fibres amyloïdes correspond à la formation de la structure native de ces protéines au même titre, par exemple, que le collagène forme spontanément des fibres structurées en hélices.

Dans le cas des protéines à domaine prion, il est donc possible de déterminer une structure à l'échelle atomique du domaine prion au sein des fibres amyloïdes, ce qui n'est pas le cas lorsque la formation des fibres amyloïdes est associée à un mauvais repliement des protéines comme dans les maladies neurodégénératives précitées.

Dans ce qui précède et ce qui suit, on entend par « *peptide comprenant un domaine prion ou un dérivé de ce domaine* », un peptide qui comprend la séquence d'acides aminés d'un domaine prion ou une séquence d'acides aminés qui dérive de la séquence d'acides aminés d'un domaine prion par une ou plusieurs modifications du type addition(s), insertion(s), délétion(s) et/ou substitution(s) d'un ou plusieurs résidus d'acide aminé, pour autant que cette ou ces modifications n'altèrent pas la capacité de ladite séquence à former des fibres amyloïdes. Les dérivés peuvent également résulter d'une ou plusieurs modifications post-traductionnelles et/ou modifications chimiques telles qu'une acétylation, une glycosylation, une amidation, une acylation, une méthylation pour autant que cette ou ces modifications n'altèrent pas la capacité de ladite séquence à former des fibres amyloïdes.

Les additions et les délétions envisagées peuvent concerner la partie C-terminale et/ou la partie N-terminale de la séquence d'acides aminés du domaine prion. Ces additions peuvent notamment consister en l'addition d'une courte séquence peptidique telle qu'une séquence composée de six résidus histidine, utile pour la purification du peptide si celui-ci est obtenu par des procédés de recombinaison génétique. A l'instar des insertions, les délétions envisagées peuvent aussi porter sur un ou plusieurs résidus d'acide aminé situés à l'intérieur de la séquence d'acides aminés.

Les substitutions envisagées peuvent être des substitutions entre acides aminés équivalents, c'est-à-dire entre des acides aminés qui présentent des propriétés chimiques ou physiques similaires (taille, charge ou polarité). Toutefois, il peut également s'agir de substitutions entre acides aminés non équivalents, c'est-à-dire entre acides aminés ne présentant pas d'homologie structurale, pour autant que, comme précédemment indiqué, ces substitutions n'affectent pas la capacité de la séquence à former des fibres amyloïdes.

Par ailleurs, ces substitutions peuvent aussi bien être réalisées avec des acides aminés du code génétique, des acides aminés naturels mais rares comme l'hydroxyproline, l'hydroxylysine, l'allohydroxy-lysine, la N-méthylglycine ou la N-éthylglycine, qu'avec des acides aminés synthétiques comme l'ornithine, la norleucine, la norvaline ou la cyclohexylalanine.

Dans le cadre de la présente invention, un dérivé d'un domaine prion comprend une séquence d'acides aminés qui présente au moins 75% et, de préférence, au moins 95% d'identité avec la séquence d'acides aminés du domaine prion dont il est dérivé, et présente une capacité à former des fibres amyloïdes.

Dans ce qui précède et ce qui suit, le membre de phrase « *qui est fonctionnalisée par des peptides transporteurs d'électrons* » signifie que la fibre amyloïde doit être vue comme constituant l'armature ou le squelette du nanofil électroconducteur et que les peptides transporteurs d'électrons doivent être vus comme des nodules qui sont fixés sur cette armature par des liaisons covalentes.

Le membre de phrase « *comprenant chacun des acides aminés liés à un ou plusieurs atomes de fer »* signifie, quant à lui, que chaque peptide transporteur d'électrons comporte des acides aminés qui sont liés directement à un ou plusieurs atomes de fer par des liaisons de coordination établies entre ces acides aminés et cet ou ces atomes de fer.

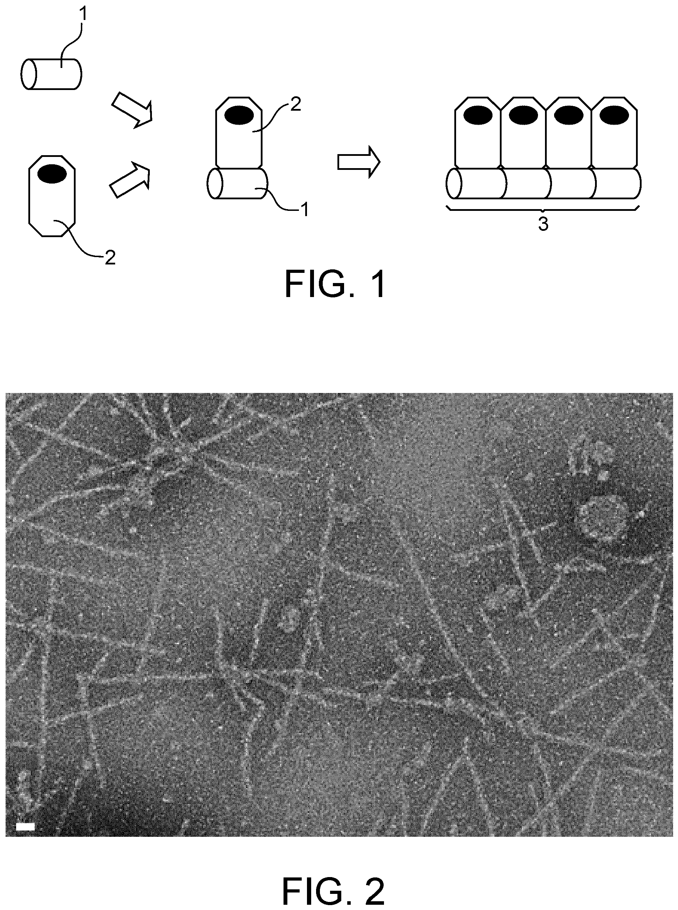

Dans le nanofil électroconducteur selon l'invention, chaque peptide comprenant un domaine prion ou un dérivé de ce domaine est, de préférence, fonctionnalisé par - et donc lié à - un peptide transporteur d'électrons, de sorte que les peptides transporteurs d'électrons puissent être régulièrement disposés sur la longueur de la fibre amyloïde et que soit, ainsi, évitée la survenue d'une rupture dans le transfert des électrons qui s'effectue le long de cette fibre. La structure ainsi obtenue est illustrée schématiquement sur la figure 1 jointe en annexe, dans laquelle chaque peptide comprenant un domaine prion ou un dérivé de ce domaine est référencé 1, chaque peptide transporteur d'électrons est référencé 2 tandis que la fibre amyloïde résultant de l'auto-assemblage des peptides comprenant un domaine prion ou un dérivé de ce domaine est référencée 3.

Conformément à l'invention, les peptides transporteurs d'électrons sont des peptides issus d'une protéine à centre fer-soufre.

Dans ce qui précède et ce qui suit, on entend par « *peptide issu d'une protéine à centre fer-soufre* », un peptide dont la séquence d'acides aminés comprend la séquence d'acides aminés d'une protéine à centre fer-soufre, ou une séquence d'acides aminés qui dérive de cette séquence par une ou plusieurs modifications du type addition(s), insertion(s), modification(s) post-traductionnelle(s) et/ou chimique(s), délétion(s) et/ou substitution(s) d'un ou plusieurs résidus d'acide aminé, pour autant que cette ou ces modifications n'altèrent pas la capacité de ladite séquence à transporter des électrons.

Les additions, les insertions, les modifications post-traductionnelles et/ou chimiques, les délétions et les substitutions envisagées sont du même type que celles précédemment évoquées.

On rappelle que les protéines à centre fer-soufre sont des protéines qui lient un cofacteur composé d'un atome de fer ou d'atomes de fer et de soufre, par l'intermédiaire des atomes de soufre de résidus cystéine, des atomes d'azote de résidus histidine, des atomes d'oxygène de résidus sérine ou de résidus acide aspartique.

Ainsi, les protéines à centre fer-soufre ne doivent pas être confondues avec les hémoprotéines ou protéines à hème, telles que le cytochrome C, qui lient également un cofacteur métallique mais dans lesquelles ce cofacteur est composé d'un noyau non protéinique, du type porphyrine, auquel est cordonné un atome de fer.

Les protéines à centre fer-soufre sont présentes chez tous les êtres vivants et servent à différentes fonctions dont le transfert d'électrons (Blondin et Girerd, Chemical Reviews 1990, 90(8), pages 1359-1376, **[5]**). Leur potentiel d'oxydoréduction va typiquement de -500 mV à plus de 500 mV.

Les centres fer-soufre les plus souvent rencontrés chez les êtres vivants sont les centres [Fe], [2Fe-2S], [3Fe-4S] et [4Fe-4S] ci-après :

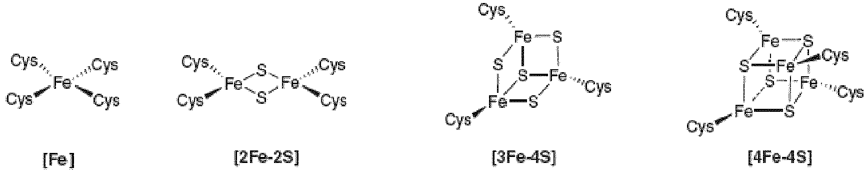

Les centres [Fe] se rencontrent le plus souvent dans des protéines de faible poids moléculaire (c'est-à-dire au plus de 6 kDa), qui sont dénommées rubrédoxines et qui sont présentes chez des bactéries anaérobies telles que les bactéries du genre *Methanococcus* ou *Desulfovibrio,* tandis que les centres [2Fe-2S], [3Fe-4S] et [4Fe-4S] se rencontrent le plus souvent dans des protéines de poids moléculaire plus élevé (typiquement de 6 à 15 kDa) telles que les ferrédoxines et les hydrogénases, et qui sont, elles, présentes chez des bactéries anaérobies comme *Clostridium pasteurianum* mais aussi chez des algues, des végétaux supérieurs et chez des vertébrés (mammifères notamment).

Aussi, la protéine dont sont issus les peptides transporteurs d'électrons est-elle, de préférence, choisie parmi les rubrédoxines, les ferrédoxines et les hydrogénases.

Parmi celles-ci, préférence est donnée aux rubrédoxines et, en particulier, à la rubrédoxine de *Methanococcus voltae,* qui présente la séquence d'acides aminés suivants :
MAIWQCTVCGYKYDEDKEKKKFEDLPADCKCPVCGAKKEMFKKL (SEQ ID NO: 1).

Cette rubrédoxine présente, en effet, l'avantage d'être de petite taille (PM = 5 kilodaltons) et d'avoir un potentiel isoélectrique proche de 7. Cette neutralité globale permet de limiter les forces de répulsion entre les peptides transporteurs d'électrons sur la fibre amyloïde et d'éviter, ainsi, que les centres fer de ces peptides ne s'éloignent les uns des autres.

Elle présente, de plus, une très grande stabilité même dans des conditions dénaturantes pour la quasi-totalité des protéines (urée à 8 moles/L ou pH < 3). Ceci est particulièrement intéressant dans le cas où les nanofils électroconducteurs selon l'invention sont préparés par expression, dans une cellule hôte, de protéines chimères comprenant chacune un peptide comprenant un domaine prion ou un dérivé de ce domaine, et un peptide issu de cette rubrédoxine, puis formation de fibres amyloïdes par auto-assemblage des peptides comprenant un domaine prion ou un dérivé de ce domaine que comportent ces protéines chimères.

En effet, grâce à cette stabilité, il est possible de purifier les protéines chimères ainsi exprimées dans des conditions dans lesquelles les peptides issus de la rubrédoxine sont fonctionnels alors que les peptides comprenant un domaine prion ou un dérivé de ce domaine sont dénaturés et d'induire ensuite la formation des fibres amyloïdes en plaçant lesdites protéines chimères dans un environnement renaturant pour les peptides issus de la rubrédoxine.

On s'affranchit, ainsi, d'une étape de renaturation des peptides issus de la rubrédoxine et de reconstitution de ses centre fer-soufre, sachant que cette étape est généralement compliquée à réaliser et nécessite de disposer d'équipements très spécifiques comme des boîtes à gants. Il en résulte un bénéfice non négligeable dans la perspective d'une production industrielle des nanofils.

Des peptides transporteurs d'électrons issus de la rubrédoxine de *Methanococcus voltae* comprennent, de préférence, la séquence d'acides aminés SEQ ID NO: 1 ou une séquence d'acides aminés qui dérive de cette séquence par une ou plusieurs modifications du type addition(s), insertion(s), modification(s) post-traductionnelle(s) et/ou chimique(s), délétion(s) et/ou substitution(s) d'un ou plusieurs résidus d'acide aminé, pour autant que cette ou ces modifications ne portent pas sur les résidus cystéine impliqués dans le centre fer-soufre de la rubrédoxine, c'est-à-dire les résidus cystéine situés en positions 6, 9, 31 et 34 de la séquence ID NO: 1, et, par conséquent, n'affectent pas la fonctionnalité de ce centre.

Typiquement, les peptides qui comprennent une séquence d'acides aminés ainsi dérivée de la séquence ID NO: 1 comprennent une séquence d'acides aminés qui présente au moins 25%, de préférence au moins 50% et, mieux encore, au moins 75% d'identité avec cette séquence.

A cet égard, on rappelle que l'identité d'une séquence par rapport à une séquence de référence s'apprécie en fonction du pourcentage de résidus d'acides aminés qui sont identiques, lorsque les deux séquences sont alignées, de manière à obtenir le maximum de correspondance entre elles. Ce pourcentage est obtenu après mise en oeuvre du meilleur alignement (alignement optimum) entre les deux séquences. L'homme du métier connaît différentes techniques permettant d'obtenir un tel pourcentage d'identité et impliquant des algorithmes d'homologie ou des programmes d'ordinateur tels que le programme BLAST.

Le pourcentage d'identité est statistique et les différences entre les deux séquences sont réparties aléatoirement le long de ces séquences. Cette définition s'applique par analogie aux séquences nucléotidiques.

Des peptides qui comprennent une séquence d'acides aminés dérivée de la séquence SEQ ID NO: 1 sont, par exemple, des peptides qui comprennent :
- la séquence d'acides aminés suivant :
   MAIWQCTVCGYKYDEDKEKKKFEDLPADYKCPVCGAKKEMFKKL (SEQ ID NO: 2),
   qui diffère de la séquence d'acides aminés SEQ ID NO: 1 en ce que le résidu cystéine situé en position 29 de cette séquence a été remplacé par un résidu tyrosine (les résidus cystéine non appariés étant, en effet, connus pour avoir un effet déstabilisant sur les centres fer-soufre) ; ou
- la séquence d'acides aminés suivante :
   AIWQCTVCGYKYDEDKEKKKFEDLPADCKCPVCGAKKEMFKKL (SEQ ID NO: 3),
   qui diffère de la séquence d'acides aminés SEQ ID NO: 1 en ce que le résidu méthionine N-terminal de cette séquence a été supprimé ; ou
- la séquence d'acides aminés suivante :
   IWQCTVCGYKYDEDKEKKKFEDLPADCKCPVCGAKKEMFKKL (SEQ ID NO: 4),
   qui diffère de la séquence d'acides aminés SEQ ID NO: 3 en ce que le résidu alanine N-terminal de cette séquence a été supprimé ; ou
- la séquence d'acides aminés suivante :
   MAIWQCTVCGYKYDEDKEKKKFEDLPADCKCPVCGAKKEMFKK (SEQ ID NO: 5),
   qui diffère de la séquence d'acides aminés SEQ ID NO: 1 en ce que le résidu leucine C-terminal de cette séquence a été supprimé ; ou
- la séquence d'acides aminés suivant :
   MAIWQCTVCGYKYDEDKEKKKFEDLPADYKCPVCGAKKEMFKKS (SEQ ID NO: 6),
   qui diffère de la séquence d'acides aminés SEQ ID NO: 2 en ce que le résidu leucine C-terminal de cette séquence a été remplacé par un résidu sérine ; ou
- la séquence d'acides aminés suivante :
   AIWQCTVCGYKYDEDKEKKKFEDLPADYKCPVCGAKKEMFKKS (SEQ ID NO: 7),
   qui diffère de la séquence d'acides aminés SEQ ID NO: 1 à la fois en ce que le résidu méthionine N-terminal a été supprimé, le résidu cystéine situé en position 29 a été remplacé par un résidu tyrosine, et en ce que le résidu leucine C-terminal a été remplacé par un résidu sérine.

Conformément à l'invention, les peptides comprenant un domaine prion ou un dérivé de ce domaine sont, de préférence, issus de la protéine Het-s, qui est une protéine prion du champignon filamenteux *Podospora anserina.*

Cette protéine est constituée de 289 résidus d'acides aminés et est impliquée dans la fonction de reconnaissance du soi et du non-soi (Dos Reis et al., Journal of Biological Chemistry 2002, 277(8), pages 5703-5706, **[6]** ; Balguerie et al., Embo Journal 2003, 22(9), pages 2071-2081, **[7]** ; Maddelein et al., 2002, Proceedings of the National Academy of Sciences of the United States of America, 2002, 99(11), pages 7402-7407 **[8]**).

Les résidus 1 à 217 contiennent la fonction de reconnaissance proprement dite tandis que les résidus 218 à 289 sont responsables de la formation des fibres amyloïdes qui sont le support de la fonction de reconnaissance.

Aussi, les peptides comprenant un domaine prion ou un dérivé de ce domaine comprennent-ils, de préférence, la séquence d'acides aminés suivants : qui correspond aux résidus 218 à 289 de la protéine Het-s, ou une séquence d'acides aminés qui dérive de cette séquence par une ou plusieurs modifications du type addition(s), insertion(s), modification(s) post-traductionnelle(s) et/ou chimique(s), délétion(s) et/ou substitution(s) d'un ou plusieurs résidus d'acide aminé, pour autant que cette ou ces modifications ne portent pas sur des résidus d'acide aminé impliqués dans la stabilité de cette séquence.

En l'espèce, les résidus d'acide aminé impliqués dans la stabilité de la séquence d'acides aminés SEQ ID NO: 8 sont les suivants :
- résidus 9 à 17 de la séquence SEQ ID NO: 8 correspondant aux résidus 226 à 234 de la protéine Het-s ;
- résidus 20 à 27 de la séquence SEQ ID NO: 8 correspondant aux résidus 237 à 244 de la protéine Het-s ;
- résidus 45 à 54 de la séquence SEQ ID NO: 8 correspondant aux résidus 262 à 271 de la protéine Het-s ;
- résidus 56 à 65 de la séquence SEQ ID NO: 8 correspondant aux résidus 273 à 282 de la protéine Het-s ;
- résidus 9 et 45 de la séquence SEQ ID NO: 8 correspondant aux résidus 226 et 262 de la protéine Het-s ;
- résidus 12 et 48 de la séquence SEQ ID NO: 8 correspondant aux résidus 229 et 265 de la protéine Het-s ;
- résidus 17 et 53 de la séquence SEQ ID NO: 8 correspondant aux résidus 234 et 270 de la protéine Het-s ;
- résidus 19 et 55 de la séquence SEQ ID NO: 8 correspondant aux résidus 236 et 272 de la protéine Het-s ; et
- résidus 11, 14, 20, 22, 24, 47, 50, 58 et 60 de la séquence SEQ ID NO: 8 correspondant aux résidus 228, 231, 237, 239, 241, 264, 267, 275 et 277 de la protéine Het-s.

Là également, les peptides qui comprennent une séquence d'acides aminés ainsi dérivée de la séquence d'acides aminés SEQ ID NO: 8 comprennent une séquence d'acides aminés qui présente au moins 75% et, de préférence, au moins 95% d'identité avec cette séquence.

Des peptides qui comprennent une séquence d'acides aminés dérivée de la séquence SEQ ID NO: 8 sont, par exemple, le peptide qui comprend la séquence d'acides aminés suivants : qui diffère de la séquence d'acides aminés SEQ ID NO: 8 en ce que le résidu lysine N-terminal de cette séquence a été remplacé par un résidu acide glutamique et en ce qu'un résidu méthionine a été ajouté en N-terminal, et les peptides qui comprennent une séquence d'acides aminés qui dérive de la SEQ ID NO: 13 par une ou plusieurs modifications du type addition(s), insertion(s), modification(s) post-traductionnelle(s) et/ou chimique(s), délétion(s) et/ou substitution(s) d'un ou plusieurs résidus d'acide aminé.
En variante, les peptides comprenant un domaine prion ou un dérivé de ce domaine peuvent également être issus d'une protéine prion autre que Het-s comme, par exemple, une protéine Sup35 ou Ure2p qui sont deux protéines prion de levures pour lesquelles la formation de fibres amyloïdes est nécessaire à leur fonction.

De manière particulièrement préférée, le nanofil électroconducteur selon l'invention est constitué d'une fibre amyloïde qui résulte de l'auto-assemblage de peptides comprenant la séquence d'acides aminés SEQ ID NO: 13 précitée, chaque peptide de cet assemblage étant fonctionnalisé par - et donc lié à - un peptide comprenant la séquence d'acides aminés SEQ ID NO: 6 précitée.

De manière également particulièrement préférée, le nanofil électroconducteur selon l'invention a une épaisseur de 5 à 10 nm et, mieux encore, de l'ordre de 8 nm et une longueur allant de 100 nm à 10 µm et, mieux encore, de 200 nm à 2 µm.

Le nanofil électroconducteur selon l'invention peut être fabriqué par des procédés consistant à greffer sur une fibre amyloïde des peptides transporteurs d'électrons, comprenant chacun un ou plusieurs acides aminés liés à un ou plusieurs atomes de fer.

Toutefois, dans le cas où chaque peptide comprenant un domaine prion ou un dérivé de ce domaine que comporte le nanofil est fonctionnalisé par un peptide transporteur d'électrons, ce nanofil peut avantageusement être fabriqué par des procédés classiquement employés pour la production de protéines recombinantes et, en particulier, par un procédé comprenant :
a) la transformation d'une cellule hôte à l'aide d'un vecteur d'expression comprenant un polynucléotide qui code une protéine chimère comprenant un peptide comprenant un domaine prion ou un dérivé de ce domaine, ce dérivé étant capable de former des fibres amyloïdes et ayant une séquence d'acides aminés qui présente au moins 75% d'identité avec la séquence d'acides aminés du domaine prion dont il est dérivé, et un peptide transporteur d'électrons, comprenant un ou plusieurs acides aminés liés à un ou plusieurs atomes de fer, le peptide transporteur d'électrons étant issu d'une protéine à centre fer-soufre ;
b) la mise en culture de la cellule hôte dans un milieu de culture ;
c) la récupération, à partir de la cellule hôte, des protéines chimères exprimées par le polynucléotide ; et
d) la formation du nanofil par assemblage des peptides issus de la protéine à domaine prion que comprennent les protéines chimères ainsi récupérées.

Aussi, l'invention a-t-elle également pour objet un procédé de fabrication d'un nanofil électroconducteur tel que précédemment défini, qui comprend les étapes a) à d) précitées.

Conformément à ce qui a été dit précédemment, le peptide transporteur d'électrons, qui est présent dans la protéine chimère, est un peptide issu d'une protéine à centre fer-soufre et, mieux encore, un peptide qui est issu d'une protéine choisie parmi les rubrédoxines, les ferrédoxines et les hydrogénases.

Auquel cas, on préfère que ce peptide soit issu d'une rubrédoxine et, de préférence, de la rubrédoxine de *Methanococcus voltae* et, en particulier, qu'il comprenne la séquence d'acides aminés SEQ ID NO: 1 précitée ou une séquence d'acides aminés qui dérive de cette séquence par une ou plusieurs modifications du type addition(s), insertion(s), modification(s) post-traductionnelle(s) et/ou chimique(s), délétion(s) et/ou substitution(s) d'un ou plusieurs résidus d'acide aminé, pour autant que cette ou ces modifications ne portent pas sur les résidus cystéine impliqués dans le centre fer-soufre de la rubrédoxine.

Par ailleurs, on préfère que le peptide comprenant un domaine prion ou un dérivé de ce domaine soit un peptide issu de la protéine prion Het-s de *Podospora anserina* et, en particulier, que ce peptide comprenne la séquence d'acides aminés SEQ ID NO: 13 précitée ou une séquence d'acides aminés qui dérive de cette séquence par une ou plusieurs modifications du type addition(s), insertion(s), modification(s) post-traductionnelle(s) et/ou chimique(s), délétion(s) et/ou substitution(s) d'un ou plusieurs résidus d'acide aminé, pour autant que cette ou ces modifications ne portent pas sur des résidus d'acide aminé impliqués dans la stabilité de cette séquence.

Tout particulièrement, on préfère que la protéine chimère comprenne un peptide comprenant la séquence d'acides aminés SEQ ID NO: 13 précitée et un peptide comprenant la séquence d'acides aminés SEQ ID NO: 6 précitée.

Ainsi, par exemple, cette protéine chimère comprend ou est constituée par la séquence d'acides aminés suivante :

L'invention a, encore, pour objets une protéine chimère telle que précédemment définie ainsi qu'un polynucléotide qui comprend une séquence nucléotidique codant cette protéine chimère.

Comme connu en soi, ce polynucléotide peut être aussi bien un ADN simple brin, un ADN double brin ou un ARN.

L'invention a, de plus, pour objets un vecteur d'expression contenant un polynucléotide selon l'invention, ce vecteur d'expression étant notamment utile pour transformer une cellule hôte et exprimer la protéine chimère selon l'invention dans cette cellule, ainsi qu'une cellule hôte comprenant ce vecteur d'expression.

Comme connu en soi, le vecteur d'expression selon l'invention comprend, outre le polynucléotide codant la protéine chimère, un ou plusieurs éléments qui permettent l'expression de cette protéine chimère dans la cellule hôte.

A titre d'exemples de tels éléments, on peut citer un promoteur constitutif ou inductible, un signal d'initiation de la transcription, un signal de terminaison de la transcription, une séquence d'initiation de la traduction, un signal de fin de la traduction.

Ce vecteur est avantageusement choisi parmi les plasmides, les cosmides, les bactériophages et les virus tels qu'un baculovirus. De tels vecteurs sont bien connus de l'homme du métier et largement décrits dans la littérature.

Quant à la cellule hôte, elle peut être tout organisme uni- ou pluricellulaire, inférieur ou supérieur, susceptible d'être transformé par un vecteur d'invention selon l'invention.

Ainsi, il peut notamment s'agir d'une levure, d'une bactérie comme *Escherichia coli,* d'un champignon, d'une cellule végétale, d'une cellule d'insecte, voire d'une cellule de mammifère à l'exception d'une cellule humaine.

Compte tenu de leurs propriétés remarquables, les nanofils électroconducteurs selon l'invention peuvent trouver de très nombreuses applications.

Ainsi, par exemple, dans la mesure où on peut prévoir avec certitude que la conduction électrique de ces nanofils sera modifiée par des interactions avec des espèces chimiques ou biologiques, ces nanofils sont susceptibles d'être utilisés dans des capteurs dévolus à la détection d'espèces chimiques ou biologiques.

Dans ce contexte, il est notamment possible d'envisager une fonctionnalisation des nanofils électroconducteurs selon l'invention par une enzyme dont l'activité produit des électrons comme, par exemple, la glucose oxydase ou la cholestérol estérase, ce qui permettrait alors aux capteurs de détecter la présence de glucose ou de cholestérol avec une très grande sensibilité puisque les électrons produits par l'activité de l'enzyme seraient alors collectés par les nanofils.

Ce type d'approche pourrait également être mis à profit pour utiliser les nanofils électroconducteurs selon l'invention dans des nanobatteries. En effet, les électrons produits par l'activité d'une enzyme pourraient servir à alimenter une nano-batterie qui serait alors constituée par des nanofils électroconducteurs selon l'invention et, donc, biodégradable.

De même, au lieu de lier une protéine délivrant un électron à partir d'un substrat, on peut envisager de lier une protéine ou molécule photosensible, délivrant un électron suite à une excitation lumineuse. Cela pourrait ainsi être utilisé pour convertir de l'énergie solaire en électricité.

Par ailleurs, l'un des défauts des polymères conducteurs étant qu'ils ne sont pas biodégradables, des nanofils selon l'invention sont susceptibles de remplacer ces polymères conducteurs dans toutes leurs utilisations macroscopiques. Ainsi, par exemple, des nanofils électroconducteurs selon l'invention pourraient être utilisés pour la fabrication de systèmes de radio-identification, encore connus sous le terme de systèmes RFID (de « **R**adio **F**requency **ID**entification ») tels que des radio-étiquettes.

Enfin, l'électronique moléculaire cherchant à mettre en application des phénomènes de transfert d'électrons à l'échelle de la molécule et, notamment, le transfert d'électrons unique, les nanofils électroconducteurs selon l'invention sont susceptibles d'être utilisés dans toutes les applications de l'électronique moléculaire.

D'autres caractéristiques et avantages de l'invention apparaîtront du complément de description qui suit et qui se rapporte à un exemple de réalisation de nanofils électroconducteurs selon l'invention ainsi qu'à un exemple portant sur la caractérisation de ces nanofils et la mise en évidence de leurs propriétés.

Bien entendu, ce complément de description n'est donné qu'à titre d'illustration de l'invention et n'en constitue en aucun cas une limitation.

### BRÈVE DESCRIPTION DES DESSINS

La figure 1, déjà commentée, illustre schématiquement une structure préférée d'un nanofil électroconducteur selon l'invention.
La figure 2 représente une image prise en microscopie électronique en transmission par coloration négative de nanofils électroconducteurs selon l'invention.

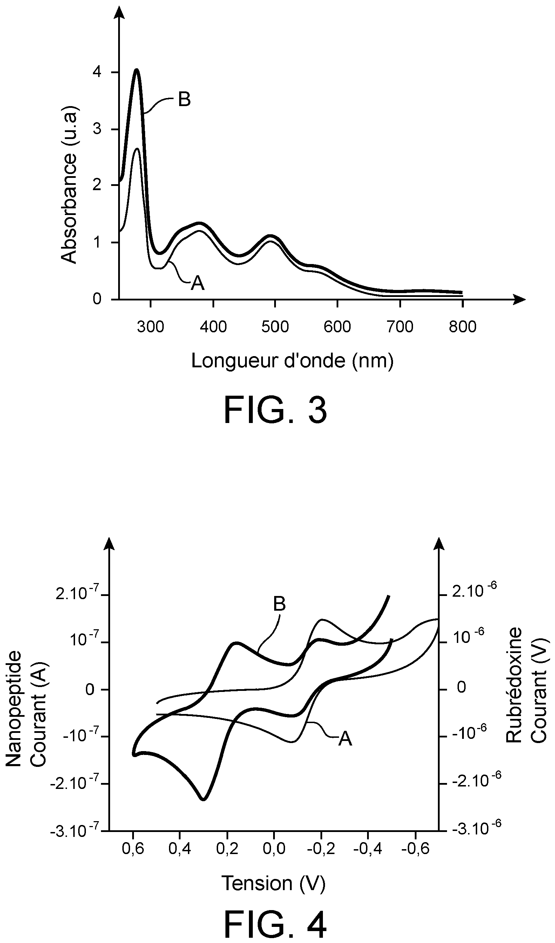

La figure 3 représente le spectre d'absorbance UV-visible (exprimée en unités arbitraires) de la rubrédoxine de *Methanococcus voltae* (spectre A) et celui de nanofils électroconducteurs selon l'invention, obtenus par expression de la protéine chimère comprenant la séquence d'acides aminés SEQ ID NO: 5 précitée dans des cellules d'*Escherichia coli* (spectre B).
La figure 4 représente les voltamogrammes enregistrés en voltamétrie cyclique pour la rubrédoxine de *Methanococcus voltae* (voltamogramme A) et pour des nanofils selon l'invention, obtenus par expression de protéines chimères comprenant chacune la séquence d'acides aminés SEQ ID NO: 5 précitée dans des cellules d'*Escherichia coli* (voltamogramme B).

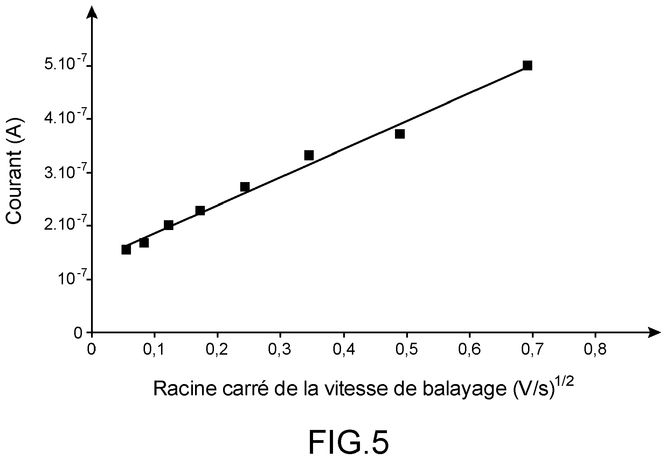

La figure 5 représente les variations de courant du pic de réduction (exprimé en ampères) en fonction de la racine carré de la vitesse de balayage (exprimée en (V/s)^{1/2}), telles qu'enregistrées en voltamétrie cyclique pour des nanofils selon l'invention, obtenus par expression de protéines chimères comprenant chacune la séquence d'acides aminés SEQ ID NO: 5 précitée dans des cellules d'*Escherichia coli.*

### EXPOSÉ DÉTAILLÉ D'UN MODE DE RÉALISATION PARTICULIER

### Exemple 1 : Réalisation de nanofils électroconducteurs selon l'invention

On réalise des nanofils électroconducteurs par expression, dans des cellules d'*Escherichia coli,* de protéines chimères comprenant chacune la séquence d'acides aminés SEQ ID NO: 9 précitée, à laquelle a été ajoutée, du côté du résidu asparagine C-terminal de cette séquence, une étiquette composée de 6 résidus histidine destinée à permettre la purification par chromatographie d'affinité sur métal immobilisé, plus connue sous le sigle IMAC (de « **I**mmobilized **Me**tal **A**ffinity **C**hromatography »), et, plus spécifiquement sur une colonne de nickel, des protéines chimères ainsi exprimées.

Les protéines chimères présentent donc la séquence d'acides aminés suivants :

Pour ce faire, la séquence nucléotidique codant pour le peptide à domaine prion : est extraite du génome de l'organisme d'origine par PCR, et introduite dans le vecteur d'expression pt7-7 entre les site de restriction NcoI et HindIII.

Ensuite, la séquence codant pour le peptide d'électroconduction : est obtenue par voie de synthèse chimique et introduite dans le même vecteur d'expression pt7-7 entre les sites de restriction NdeI et NcoI.

L'introduction de séquences dans un vecteur d'expression se fait via les sites de restriction précédemment énoncés. Les sites de restriction ont la particularité de présenter une séquence dite palindromique (le brin complémentaire renversé à la même séquence) de 4 à 8 nucléotides. Cette séquence peut être clivée spécifiquement par l'enzyme de restriction du même nom et liguée (s'il y a complémentarité des séquence) par une ligase. La T4 DNA ligase a été utilisée dans le présent exemple.

Le vecteur d'expression ainsi modifié est introduit à son tour dans des cellules d'*Escherichia coli BL21 (DE3) PlysS.*

L'introduction du vecteur dans les cellules est ce qu'on appelle une transformation. Elle est effectuée de la manière suivants :
- ajout du plasmide dans le milieu extra-cellulaire ;
- incubation d'une durée de 30 à 60 minutes à 4°C ;
- choc thermique d'une durée de 30 secondes à 2 minutes à 42°C ;
- 15 minutes à 4°C ;
- étalement sur boites LB agar pour sélectionner les bactéries résistante à l'antibiotique (c'est-à-dire ayant intégré le vecteur d'expression).

Les cellules d'*Escherichia coli* ainsi transformées sont ensuite cultivées dans un milieu minimum enrichi en fer 50 µmol/L jusqu'à ce que la densité optique (DO) atteigne une valeur de 0,7. L'expression des protéines est alors induite en ajoutant 1 mM d'imidazole dans le milieu et les bactéries sont incubées pendant 4 heures supplémentaires.

Après quoi :
- le milieu de culture est centrifugé à 4 000 rcf ;
- les culots bactériens sont remis en suspension dans un tampon tris, pH 8, contenant 8 mol/L d'urée pour inhiber la formation de fibres amyloïdes par les protéines chimères exprimées ;
- les cellules sont lysées par sonication ;
- les lysats cellulaires sont centrifugés à 40 000 rcf et filtrés à 0,45 µm pour éliminer leur partie non soluble ;
- la partie soluble des lysats cellulaires est chargée sur une colonne de nickel préalablement équilibrée avec du tampon A (tris-HCl : 10 mmol/L ; urée : 8 mol/L ; pH : 8) ;
- la colonne est lavée avec du tampon A puis avec du tampon A additionné de 25 mol/L d'imidazole ;
- la colonne est éluée avec du tampon A additionné de 300 mmol/L d'imidazole ; et
- le tampon dans lequel se trouvent les protéines chimères ainsi récupérées est dialysé contre de l'acide acétique à 1% (pH 2,7) de manière à pouvoir conserver ces protéines chimères sans qu'elles ne forment des fibres amyloïdes.

Dans ces conditions, il est possible de récupérer 8 mg de protéines chimères à partir d'un litre de culture de bactéries en erlen.

La formation des fibres amyloïdes est ensuite déclenchée par dialyse de l'acide acétique contre de l'eau.

### Exemple 2 : Caractérisation et propriétés des nanofils électroconducteurs selon l'invention

### 2.1. Structure fibrillaire des nanofils

La figure 3 montre une image prise en microscopie électronique en transmission par coloration négative au sel d'uranyle des nanofils obtenus à l'exemple 1 et sur laquelle l'échelle, qui est représentée par le rectangle situé à l'angle gauche du bas de cette image, correspond à 20 nm.

Cette figure montre que les nanofils présentent, comme attendu, une structure fibrillaire et qu'ils ont sensiblement une épaisseur de 8 nm pour une longueur allant de 200 nm à 2 µm.

### 2.2. Propriétés d'électroconduction des nanofils

Afin que les nanofils obtenus à l'exemple 1 ci-avant puissent conduire les électrons de part et d'autre de la fibre amyloïde qui les constitue, il est important que les séquences peptidiques issues de la rubrédoxine de *Methanococcus voltae,* qui fonctionnalisent cette fibre amyloïde, soient correctement repliées sur leur centre fer-soufre.

La rubrédoxine de *Methanococcus voltae* présente des raies d'absorbance UV-visible typiques à 360, 380, 490, 570 et 750 nm.

Aussi, pour vérifier que les séquences peptidiques fonctionnalisant la fibre amyloïde des nanofils sont correctement repliées sur leur centre fer-soufre et, par conséquent, fonctionnelles, on mesure donc l'absorbance de ces nanofils aux longueurs d'onde précitées.

Pour ce faire, on utilise un spectroscope UV-visible en mode balayage entre 800 et 250 nm. L'échantillon est disposé dans une cuve en quartz de chemin optique égal à 1 cm, traversée par le faisceau du spectroscope qui mesure ainsi l'absorbance de la solution protéique à toutes les longueurs d'ondes dans la gamme de balayage.

Les résultats sont montrés sur la figure 3 dans laquelle la courbe A représente le spectre d'absorbance UV-visible de la rubrédoxine de *Methano-coccus voltae* tandis que la courbe B représente le spectre d'absorbance UV-visible des nanofils.

Cette figure montre que les raies d'absorbance UV-visible des nanofils sont identiques à celles de la rubrédoxine de *Methanococcus voltae* et les nanofils, ce qui signifie que les séquences peptidiques fonctionnalisant la fibre amyloïde des nanofils sont correctement repliées sur leur centre fer-soufre et fonctionnelles.

Par ailleurs, la capacité à conduire les électrons d'un objet dans lequel cette capacité est basée sur des réactions d'oxydoréduction peut être appréciée par voltamétrie cyclique.

Le principe de cette technique est d'appliquer un potentiel, que l'on fait varier de façon cyclique, à une solution dans laquelle se trouve l'objet et de mesurer le courant fourni ou accepté par les espèces chimiques donneuses ou accepteuses d'électrons que cet objet comporte.

Cela renseigne sur le potentiel redox de ces espèces chimiques et sur leur caractère diffusif. En effet, le transfert d'électrons d'une espèce chimique à l'électrode de travail dépend de la capacité de cette espèce à diffuser sur la surface de cette électrode.

Selon la vitesse de balayage du potentiel appliqué, cette diffusion est différente et le nombre d'électrons transféré est différent. Par contre, si l'espèce chimique est adsorbée sur l'électrode de travail, elle ne diffuse pas.

Une loi permet de relier le courant électronique fourni par les espèces chimiques donneuses d'électrons à la vitesse de balayage du potentiel par l'intermédiaire du coefficient de diffusion de ces espèces.

Ceci étant précisé, lorsque l'on soumet la rubrédoxine de *Methanococcus voltae* à un test de voltamétrie cyclique (électrode de travail en carbone vitreux, électrode de référence Ag/AgCl, contre-électrode de platine, solution de rubrédoxine à 340 µmol/L, NaCl 100 mM, vitesse de balayage de 30 mV/s), on obtient le voltamogramme A de la figure 4. La rubrédoxine présente un potentiel d'oxydoréduction de -100 mV et un coefficient de diffusion de 1,5.10⁻¹⁰ m²/s.

Lorsque l'on soumet les nanofils obtenus à l'exemple 1 à un test de voltamétrie cyclique dans les mêmes conditions, on obtient le voltamogramme B montré sur la figure 4. On observe que ces nanofils sont adsorbés à la surface de l'électrode de travail. Par conséquent, les électrons ne peuvent plus être transportés jusqu'à l'électrode de travail par diffusion des séquences issues de la rubrédoxine de *Methanococcus voltae.*

Or, comme montré sur la figure 4, on voit apparaître un deuxième couple redox vers +250 mV dont le pic de réduction, comme montré sur la figure 5, garde un caractère diffusif lorsque l'on fait varier la vitesse de balayage. On ne peut calculer précisément le coefficient de diffusion car la concentration en nanofils est inconnue dans le présent test. Cependant, on sait qu'il est au moins de deux ordres de grandeur plus faible que celui de la rubrédoxine toute seule. La seule explication à ces différents phénomènes est que les électrons diffusent le long des nanofils, ce qui signifie que ces nanofils peuvent transporter un courant électrique.

### REFERENCES CITEES

[1] Jones et Grainger, Advanced Drug Delivery Reviews 2009, 61(6), pages 438-456
[2] Sipe et Cohen, Journal of Structural Biology 2000, 130(2-3), pages 88-98
[3] Sabate et al., Journal of Structural Biology 2008, 162(3), pages 387-396
[4] Baxa et al., Advances in Protein Chemistry 2006, 73, pages 125-180
[5] Blondin et Girerd, Chemical Reviews 1990, 90(8), pages 1359-1376
[6] Dos Reis et al. 2002, Journal of Biological Chemistry, 277(8), pages 5703-5706
[7] Balguerie et al. 2003, Embo Journal, 22(9), pages 2071-2081
[8] Maddelein et al., 2002, Proceedings of the National Academy of Sciences of the United States of America, 2002, 99(11), pages 7402-7407
[9] WO A 2005/118633
[10] Baldwin et al., Journal of the American Chemical Society 2006, 128, pages 2162-2163

## Revendications

1. Nanofil électroconducteur, comprenant une fibre amyloïde qui résulte de l'auto-assemblage de peptides comprenant un domaine prion ou un dérivé de ce domaine, ce dérivé étant capable de former des fibres amyloïdes et ayant une séquence d'acides aminés qui présente au moins 75% d'identité avec la séquence d'acides aminés du domaine prion dont il est dérivé, et dans lequel la fibre amyloïde est fonctionnalisée par des peptides transporteurs d'électrons comprenant chacun des acides aminés liés à un ou plusieurs atomes de fer, les peptides transporteurs d'électrons étant issus d'une protéine à centre fer-soufre.

2. Nanofil électroconducteur selon la revendication 1, dans lequel chaque peptide comprenant un domaine prion ou un dérivé de ce domaine est fonctionnalisé par un peptide transporteur d'électrons.

3. Procédé de fabrication d'un nanofil électroconducteur selon la revendication 1 ou 2, qui comprend :
a) la transformation d'une cellule hôte à l'aide d'un vecteur d'expression comprenant un polynucléotide qui code une protéine chimère comprenant un peptide comprenant un domaine prion ou un dérivé de ce domaine, ce dérivé étant capable de former des fibres amyloïdes et ayant une séquence d'acides aminés qui présente au moins 75% d'identité avec la séquence d'acides aminés du domaine prion dont il est dérivé, et un peptide transporteur d'électrons comprenant un ou plusieurs acides aminés liés à un ou plusieurs atomes de fer, le peptide transporteur d'électrons étant issu d'une protéine à centre fer-soufre ;
b) la mise en culture de la cellule hôte dans un milieu de culture ;
c) la récupération, à partir de la cellule hôte, des protéines chimères exprimées par le polynucléotide ; et
d) la formation du nanofil par les peptides comprenant un domaine prion ou un dérivé de ce domaine que comprennent les protéines chimères ainsi récupérées.

4. Nanofil électroconducteur selon la revendication 1 ou 2, ou procédé selon la revendication 3, dans lequel le(s) peptide(s) transporteur(s) d'électrons est (sont) un (des) peptide(s) issu(s) d'une protéine choisie parmi les rubrédoxines, les ferrédoxines et les hydrogénases.

5. Nanofil électroconducteur ou procédé selon la revendication 4, dans lequel le(s) peptide(s) transporteur(s) d'électrons est (sont) un (des) peptide(s) issu(s) d'une rubrédoxine.

6. Nanofil électroconducteur ou procédé selon la revendication 5, dans lequel le(s) peptide(s) transporteur(s) d'électrons est (sont) un (des) peptide(s) issu(s) de la rubrédoxine de *Methanococcus voltae.*

7. Nanofil électroconducteur ou procédé selon la revendication 6, dans lequel le(s) peptide(s) transporteur(s) d'électrons comprend (comprennent) la séquence d'acides aminés suivants :
MAIWQCTVCGYKYDEDKECKKFEDLPADYKCPVCGAKKEMFKKL (SEQ ID NO: 1),
ou une séquence d'acides aminés qui dérive de cette séquence par une ou plusieurs modifications du type addition(s), insertion(s), modification(s) post-traductionnelle(s) et/ou chimique(s), délétion(s) et/ou substitution(s) d'un ou plusieurs résidus d'acide aminé.

8. Nanofil électroconducteur selon l'une quelconque des revendications 1, 2 et 4 à 7, ou procédé selon l'une quelconque des revendications 3 à 7, dans lequel le(s) peptide(s) comprenant un domaine prion ou un dérivé de ce domaine est (sont) un (des) peptide(s) issu(s) de la protéine prion Het-s de *Podospora anserina.*

9. Nanofil électroconducteur ou procédé selon la revendication 8, dans lequel le(s) peptide(s) comprenant un domaine prion ou un dérivé de ce domaine comprend (comprennent) la séquence d'acides aminés suivante : ou une séquence d'acides aminés qui dérive de cette séquence par une ou plusieurs modifications du type addition(s), insertion(s), modification(s) post-traductionnelle(s) et/ou chimique(s), délétion(s) et/ou substitution(s) d'un ou plusieurs résidus d'acide aminé.

10. Nanofil électroconducteur selon l'une quelconque des revendications 1, 2 et 4 à 9, qui est constitué d'une fibre amyloïde qui résulte de l'auto-assemblage de peptides comprenant la séquence d'acides aminés suivante : chaque peptide de cet assemblage étant fonctionnalisé par un peptide répondant à la séquence d'acides aminés suivante :
MAIWQCTVCGYKYDEDKEKKKFEDLPADYKCPVCGAKKEMFKKS (SEQ ID NO: 6).

11. Procédé selon l'une quelconque des revendications 3 à 9, dans lequel la protéine chimère comprend un peptide comprenant la séquence d'acides aminés suivante :
MAIWQCTVCGYKYDEDKEKKKFEDLPADYKCPVCGAKKEMFKKS (SEQ ID NO: 13),
et un peptide comprenant la séquence d'acides aminés suivante :

12. Procédé selon la revendication 11, dans lequel la protéine chimère comprend ou est constituée par la séquence d'acides aminés suivants :

13. Protéine chimère, qui est telle que définie dans l'une quelconque des revendications 3 à 9, 11 et 12.

14. Polynucléotide, qui comprend une séquence nucléotidique codant une protéine chimère selon la revendication 13.

15. Vecteur d'expression, qui comprend un polynucléotide selon la revendication 14.

16. Cellule hôte, qui est transformée par un vecteur d'expression selon la revendication 15.

## Patentansprüche

1. Elektrisch leitender Nanodraht, welcher eine Amyloidfaser umfasst, die durch Selbstaufbau aus Peptiden entstanden ist, welche eine Prionendomäne oder ein Derivat einer solchen Domäne aufweisen, wobei das Derivat fähig ist, Amyloidfasern zu bilden und eine Aminosäuresequenz aufweist, welche mindestens 75 % Identität aufweist mit der Aminosäuresequenz der Prionendomäne, von welcher es abgeleitet ist, und worin die Amyloidfaser durch Elektronen transportierende Peptide funktionalisiert ist, in welchen jede der Aminosäuren verbunden mit einem oder mehreren Eisenatomen vorliegt, wobei die Elektronen transportierenden Peptide aus einem Eisen-Schwefel-Cluster-Protein gebildet sind.

2. Elektrisch leitfähiger Nanodraht gemäß Anspruch 1, worin jedes Peptid, welches eine Prionendomäne oder ein Derivat einer solchen Domäne umfasst, durch ein Elektronen transportierendes Peptid funktionalisiert ist.

3. Verfahren zur Herstellung eines elektrisch leitenden Nanodrahts gemäß den Ansprüchen 1 oder 2, welches umfasst:
a) die Transformation einer Wirtszelle mithilfe eines Expressionsvektors, welcher ein Polynukleotid umfasst, welches für ein chimäres Protein kodiert, welches ein Peptid umfasst, welches eine Prionendomäne oder ein Derivat einer solchen Domäne umfasst, wobei das Derivat zur Bildung von Amyloidfasern fähig ist und eine Aminosäuresequenz aufweist, welche mindestens 75 % Identität mit der Aminosäuresequenz der Prionendomäne, von welcher es abgeleitet ist, aufweist, und welches ein Elektronen transportierendes Peptid umfasst, welches ein oder mehrere Aminosäuren aufweist, welche an ein oder mehrere Eisenatome gebunden sind, wobei das Elektronen transportierende Peptid gebildet ist aus einem Eisen-Schwefel-Cluster-Protein;
b) das Kultivieren der Wirtszelle in einem Kulturmedium;
c) das Gewinnen der chimären Proteine, welche durch das Polynukleotid exprimiert werden aus der Wirtszelle; und
d) die Bildung des Nanodrahts aus den Peptiden, welche eine Prionendomäne oder ein Derivat einer solchen Domäne umfassen, und welche die so gewonnenen chimären Proteine enthalten.

4. Elektrisch leitender Nanodraht gemäß den Ansprüchen 1 oder 2, oder hergestellt gemäß Anspruch 3, worin das oder die Elektronen transportierende(n) Peptid(e) ein oder mehrere Peptid(e) ist oder sind, welche(s) gebildet sind (ist) aus einem Protein ausgewählt aus den Rubredoxinen, den Ferredoxinen und den Hydrogenasen.

5. Elektrisch leitender Nanodraht oder Verfahren gemäß Anspruch 4, worin das oder die Elektronen transportierende(n) Peptid(e) aus einem Rubredoxin hergestellt ist oder sind.

6. Elektrisch leitender Nanodraht oder Verfahren gemäß Anspruch 5, worin das oder die Elektronen transportierende(n) Peptid(e) ein oder mehrere Peptide ist oder sind, welche(s) aus dem Rubredoxin von *Methanococcus voltae* hergestellt wurde(n).

7. Elektrisch leitender Nanodraht oder Verfahren gemäß Anspruch 6, worin das oder die Elektronen transportierende(n) Peptid(e) die folgende Aminosäuresequenz umfasst bzw. umfassen:
MAIWQCTVCGYKYDEDKECKKFEDLPADYKCPVCGAKKEMFKKL (SEQ ID NO: 1),
oder eine Aminosäuresequenz, die von dieser Sequenz abgeleitet ist durch eine oder mehrere Modifikation(en) des Typs Addition(en), Insertion(en), posttranslationale oder/und chemische Modifikation(en), Deletion(en) und/oder Substitution(en) eines oder mehrerer Aminosäurereste(s).

8. Elektrisch leitender Nanodraht gemäß einem der Ansprüche 1, 2 und 4 bis 7, oder Verfahren gemäß einem der Ansprüche 3 bis 7, worin das (oder die) Peptid(e), welche(s) eine Prionendomäne oder ein Derivat einer solchen Domäne umfasst (umfassen), hergestellt ist (sind) aus dem Prionenprotein Het-s aus *Podospora anserina.*

9. Elektrisch leitender Nanodraht oder Verfahren gemäß Anspruch 8, worin das (die) Peptid(e) umfassend eine Prionendomäne oder ein Derivat einer solchen Domäne die folgende Aminosäursequenz umfasst (umfassen): oder eine Aminosäuresequenz, die von dieser Sequenz abgeleitet ist durch eine oder mehrere Modifikation(en) des Typs Addition(en), Insertion(en), posttranslationale oder/und chemische Modifikation(en), Deletion(en) und/oder Substitution(en) eines oder mehrere Aminosäurereste.

10. Elektrisch leitender Nanodraht gemäß einem der Ansprüche 1, 2 und 4 bis 9, welcher aufgebaut ist aus einer Amyloidfaser, die aus dem Selbstaufbau von Peptiden umfassend die folgende Aminosäuresequenz entsteht: wobei jedes Peptid dieses Aufbaus durch ein Peptid funktionalisiert ist, welches der folgenden Aminosäuresequenz entspricht:
MAIWQCTVCGYKYDEDKEKKKFEDLPADYKCPVCGAKKEMFKKS (SEQ ID NO: 6).

11. Verfahren gemäß einem der Ansprüche 3 bis 9, worin das chimäre Protein ein Peptid umfasst, welches die folgende Aminosäuresequenz aufweist: und ein Peptid, welches die folgende Aminosäuresequenz aufweist:
MAIWQCTVCGYKYDEDKEKKKFEDLPADYKCPVCGAKKEMFKKS (SEQ ID NO: 6).

12. Verfahren gemäß Anspruch 11, worin das chimäre Protein die folgende Aminosäuresequenz aufweist oder aus ihr aufgebaut ist:

13. Chimäres Protein, wie es in einem der Ansprüche 3 bis 9, 11 und 12 definiert ist.

14. Polynukleotid, welches eine Nukleinsäure aufweist, welche für ein chimäres Protein gemäß Anspruch 13 kodiert.

15. Expressionsvektor, der ein Polynukleotid gemäß Anspruch 14 umfasst.

16. Wirtszelle, welche transformiert ist durch einen Expressionsvektor gemäß Anspruch 15.

## Claims

1. Electroconducting nanowire, comprising an amyloid fibre which results from the self-assembling of peptides comprising a prion domain or a derivative of this domain, this derivative being capable of forming amyloid fibres and having a sequence of amino acids that has at least 75% identity with the sequence of amino acids of the prion domain from which it derives, and wherein the amyloid fibre is functionalized by electron transporting peptides, each comprising amino acids bound to one or more atoms of iron, the electron transporting peptides being issued from a protein with iron-sulphur centre.

2. Electroconducting nanowire according to claim 1, wherein each peptide comprising a prion domain or a derivative of this domain is functionalized by an electron transporting peptide.

3. Method for manufacturing an electroconducting nanowire according to claim 1 or 2, which comprises:
a) transforming a host cell using an expression vector comprising a polynucleotide which encodes a chimeric protein comprising a peptide comprising a prion domain or a derivative of this domain, this derivative being capable of forming amyloid fibres and having a sequence of amino acids that has at least 75% identity with the sequence of amino acids of the prion domain from which it derives, and an electron transporting peptide comprising one or more amino acids bound to one or more atoms of iron, the electron transporting peptide being issued from a protein with iron-sulphur centre;
b) culturing the host cell in a culture medium;
c) recovering, from the host cell, the chimeric proteins expressed by the polynucleotide; and
d) forming the nanowire by the peptides comprising a prion domain or a derivative of this domain that the chimeric proteins thereby recovered comprise.

4. Electroconducting nanowire according to claim 1 or 2, or method according to claim 3, wherein the electron transporting peptide(s) is (are) peptide(s) issued from a protein selected from rubredoxins, ferredoxins and hydrogenases.

5. Electroconducting nanowire or method according to claim 4, wherein the electron transporting peptide(s) is (are) peptide(s) issued from a rubredoxin.

6. Electroconducting nanowire or method according to claim 5, wherein the electron transporting peptide(s) is (are) peptide(s) issued from the rubredoxin of *Methanococcus voltae.*

7. Electroconducting nanowire or method according to claim 6, wherein the electron transporting peptide(s) comprises (comprise) the following sequence of amino acids:
MAIWQCTVCGYKYDEDKEKKKFEDLPADCKCPVCGAKKEMFKKL (SEQ ID NO: 1),
or a sequence of amino acids which derives from this sequence by one or more modifications of the type addition(s), insertion(s), post-translational and/or chemical modification(s), deletion(s) and/or substitution(s) of one or more amino acid residues.

8. Electroconducting nanowire according to any of claims 1, 2 and 4 to 7, or method according to any of claims 3 to 7, wherein the peptide(s) comprising a prion domain or a derivative of this domain is (are) peptide(s) issued from the Het-s prion protein of *Podospora anserina.*

9. Electroconducting nanowire or method according to claim 8, wherein the peptide(s) comprising a prion domain or a derivative of this domain comprises (comprise) the following sequence of amino acids: or a sequence of amino acids which derives from this sequence by one or more modifications of the type addition(s), insertion(s), post-translational and/or chemical modification(s), deletion(s) and/or substitution(s) of one or more amino acid residues.

10. Electroconducting nanowire according to any of claims 1, 2 and 4 to 9, which is constituted of an amyloid fibre which results from the self-assembling of peptides comprising the following sequence of amino acids: each peptide of this assemblage being functionalized by a peptide meeting the following sequence of amino acids:
MAIWQCTVCGYKYDEDKEKKKFEDLPADYKCPVCGAKKEMFKKS (SEQ ID NO: 6).

11. Method according to any of claims 3 to 9, wherein the chimeric protein comprises a peptide comprising the following sequence of amino acids:
MAIWQCTVCGYKYDEDKEKKKFEDLPADYKCPVCGAKKEMFKKS (SEQ ID NO: 13),
and a peptide comprising the following sequence of amino acids:

12. Method according to claim 11, wherein the chimeric protein comprises or is constituted of the following sequence of amino acids:

13. Chimeric protein, which is as defined in any of claims 3 to 9, 11 and 12.

14. Polynucleotide, which comprises a nucleotide sequence encoding a chimeric protein according to claim 13.

15. Expression vector, which comprises a polynucleotide according to claim 14.

16. Host cell, which is transformed by an expression vector according to claim 15.


### SEQUENCE LISTING

<110> Commissariat à l'Energie Atomique et aux Energies Alternatives
<120> NANOFIL ELECTROCONDUCTEUR BIODEGRADABLE, SON PROCEDE DE FABRICATION ET SES UTILISATIONS
<130> SP38606 SL
<140> PCT/EP2012/XXXXXX
   <141> 2012-03-06
<150> FR 1151851
   <151> 2011-03-07
<160> 13
<170> PatentIn version 3.5
<210> 1
   <211> 44
   <212> PRT
   <213> Methanococcus voltae
<220>
   <221> PEPTIDE
   <222> (1)..(44)
   <223> Rubrédoxine
<400> 1
   Met Ala Ile Trp Gln Cys Thr Val Cys Gly Tyr Lys Tyr Asp Glu Asp
   1               5                   10                  15
   Lys Glu Lys Lys Lys Phe Glu Asp Leu Pro Ala Asp Cys Lys Cys Pro
               20                  25                  30
   Val Cys Gly Ala Lys Lys Glu Met Phe Lys Lys Leu
           35                  40
<210> 2
   <211> 44
   <212> PRT
   <213> unidentified
<220>
   <221> PEPTIDE
   <222> (1)..(44)
   <223> Séquence dérivée de la séquence SEQ ID NO: 1
<400> 2
   Met Ala Ile Trp Gln Cys Thr Val Cys Gly Tyr Lys Tyr Asp Glu Asp
   1               5                   10                  15
   Lys Glu Lys Lys Lys Phe Glu Asp Leu Pro Ala Asp Tyr Lys Cys Pro
                   20                  25                  30
   Val Cys Gly Ala Lys Lys Glu Met Phe Lys Lys Leu
           35                  40
<210> 3
   <211> 43
   <212> PRT
   <213> unidentified
<220>
   <221> PEPTIDE
   <222> (1)..(43)
   <223> Séquence dérivée de la séquence SEQ ID NO: 1
<400> 3
   Ala Ile Trp Gln Cys Thr Val Cys Gly Tyr Lys Tyr Asp Glu Asp Lys
   1               5                   10                  15
   Glu Lys Lys Lys Phe Glu Asp Leu Pro Ala Asp Cys Lys Cys Pro Val
               20                  25                  30
   Cys Gly Ala Lys Lys Glu Met Phe Lys Lys Leu
           35                  40
<210> 4
   <211> 42
   <212> PRT
   <213> unidentified
<220>
   <221> PEPTIDE
   <222> (1)..(42)
   <223> Séquence dérivée de la séquence SEQ ID NO: 1
<400> 4
   Ile Trp Gln Cys Thr Val Cys Gly Tyr Lys Tyr Asp Glu Asp Lys Glu
   1               5                   10                  15
   Lys Lys Lys Phe Glu Asp Leu Pro Ala Asp Cys Lys Cys Pro Val Cys
               20                  25                  30
   Gly Ala Lys Lys Glu Met Phe Lys Lys Leu
           35                  40
<210> 5
   <211> 43
   <212> PRT
   <213> unidentified
<220>
   <221> PEPTIDE
   <222> (1)..(43)
   <223> Séquence dérivée de la séquence SEQ ID NO: 1
<400> 5
   Met Ala Ile Trp Gln Cys Thr Val Cys Gly Tyr Lys Tyr Asp Glu Asp
   1               5                   10                  15
   Lys Glu Lys Lys Lys Phe Glu Asp Leu Pro Ala Asp Cys Lys Cys Pro
               20                  25                  30
   Val Cys Gly Ala Lys Lys Glu Met Phe Lys Lys
           35                  40
<210> 6
   <211> 44
   <212> PRT
   <213> unidentified
<220>
   <221> PEPTIDE
   <222> (1)..(44)
   <223> Séquence dérivée de la séquence SEQ ID NO: 1
<400> 6
   Met Ala Ile Trp Gln Cys Thr Val Cys Gly Tyr Lys Tyr Asp Glu Asp
   1               5                   10                  15
   Lys Glu Lys Lys Lys Phe Glu Asp Leu Pro Ala Asp Tyr Lys Cys Pro
               20                  25                  30
   Val Cys Gly Ala Lys Lys Glu Met Phe Lys Lys Ser
           35                  40
<210> 7
   <211> 43
   <212> PRT
   <213> unidentified
<220>
   <221> PEPTIDE
   <222> (1)..(43)
   <223> Séquence dérivée de la séquence SEQ ID NO: 1
<400> 7
   Ala Ile Trp Gln Cys Thr Val Cys Gly Tyr Lys Tyr Asp Glu Asp Lys
   1               5                   10                  15
   Glu Lys Lys Lys Phe Glu Asp Leu Pro Ala Asp Tyr Lys Cys Pro Val
               20                  25                  30
   Cys Gly Ala Lys Lys Glu Met Phe Lys Lys Ser
           35                  40
<210> 8
   <211> 72
   <212> PRT
   <213> Podospora anserina
<220>
   <221> PEPTIDE
   <222> (1)..(72)
   <223> Résidus 218 à 289 de la protéine Het-s
<400> 8
   Lys Ile Asp Ala Ile Val Gly Arg Asn Ser Ala Lys Asp Ile Arg Thr
   1               5                   10                  15
   Glu Glu Arg Ala Arg Val Gln Leu Gly Asn Val Val Thr Ala Ala Ala
               20                  25                  30
   Leu His Gly Gly Ile Arg Ile Ser Asp Gln Thr Thr Asn Ser Val Glu
           35                  40                  45
   Thr Val Val Gly Lys Gly Glu Ser Arg Val Leu Ile Gly Asn Glu Tyr
       50                  55                  60
   Gly Gly Lys Gly Phe Trp Asp Asn
   65                  70
<210> 9
   <211> 117
   <212> PRT
   <213> unidentified
<220>
   <221> PEPTIDE
   <222> (1)..(117)
   <223> Protéine chimère formée par les séquences SEQ ID NO: 6 et SEQ ID NO: 13
<400> 9
   Met Ala Ile Trp Gln Cys Thr Val Cys Gly Tyr Lys Tyr Asp Glu Asp
   1               5                   10                  15
   Lys Glu Lys Lys Lys Phe Glu Asp Leu Pro Ala Asp Tyr Lys Cys Pro
               20                  25                  30
   Val Cys Gly Ala Lys Lys Glu Met Phe Lys Lys Ser Met Glu Ile Asp
           35                  40                  45
   Ala Ile Val Gly Arg Asn Ser Ala Lys Asp Ile Arg Thr Glu Glu Arg
       50                  55                  60
   Ala Arg Val Gln Leu Gly Asn Val Val Thr Ala Ala Ala Leu His Gly
   65                  70                  75                  80
   Gly Ile Arg Ile Ser Asp Gln Thr Thr Asn Ser Val Glu Thr Val Val
                   85                  90                  95
   Gly Lys Gly Glu Ser Arg Val Leu Ile Gly Asn Glu Tyr Gly Gly Lys
               100                 105                 110
   Gly Phe Trp Asp Asn
           115
<210> 10
   <211> 123
   <212> PRT
   <213> unidentified
<220>
   <221> PEPTIDE
   <222> (1)..(123)
   <223> Protéine chimère dérivée de la séquence SEQ ID NO: 9
<400> 10
   Met Ala Ile Trp Gln Cys Thr Val Cys Gly Tyr Lys Tyr Asp Glu Asp
   1               5                   10                  15
   Lys Glu Lys Lys Lys Phe Glu Asp Leu Pro Ala Asp Tyr Lys Cys Pro
               20                  25                  30
   Val Cys Gly Ala Lys Lys Glu Met Phe Lys Lys Ser Met Glu Ile Asp
           35                  40                  45
   Ala Ile Val Gly Arg Asn Ser Ala Lys Asp Ile Arg Thr Glu Glu Arg
       50                  55                  60
   Ala Arg Val Gln Leu Gly Asn Val Val Thr Ala Ala Ala Leu His Gly
   65                  70                  75                  80
   Gly Ile Arg Ile Ser Asp Gln Thr Thr Asn Ser Val Glu Thr Val Val
                   85                  90                  95
   Gly Lys Gly Glu Ser Arg Val Leu Ile Gly Asn Glu Tyr Gly Gly Lys
               100                 105                 110
   Gly Phe Trp Asp Asn His His His His His His
           115                 120
<210> 11
   <211> 240
   <212> DNA
   <213> unidentified
<220>
   <221> misc-feature
   <222> (1)..(240)
   <223> Séquence nucléotidique codant la séquence SEQ ID NO: 13
<400> 11
   atggagatcg acgcgattgt gggaaggaat tccgccaagg atatcagaac agaggaacgt      60
   gcaagggtcc agctcggcaa tgttgtcact gcggcagccc tacatggtgg gatccgtatc     120
   agcgaccaga caaccaactc ggtagagacg gttgtgggga agggcgagtc tagggtcctt     180
   attggaaatg agtacggagg taaggggttc tgggataatc accatcacca tcatcactaa     240
<210> 12
   <211> 132
   <212> DNA
   <213> unidentified
<220>
   <221> misc-feature
   <222> (1)..(132)
   <223> Séquence nucléotidique codant la séquence SEQ ID NO: 6
<400> 12
   atggcgatct ggcagtgtac cgtttgcggt tacaaatacg atgaagataa agaaaagaaa      60
   aaatttgaag acctgccggc ggattacaaa tgtccggttt gcggtgcgaa aaaagaaatg     120
   tttaagaaat cc                                                         132
<210> 13
   <211> 73
   <212> PRT
   <213> unidentified
<220>
   <221> PEPTIDE
   <222> (1)..(73)
   <223> Séquence dérivée de la séquence SEQ ID NO: 8
<400> 13
   Met Glu Ile Asp Ala Ile Val Gly Arg Asn Ser Ala Lys Asp Ile Arg
   1               5                   10                  15
   Thr Glu Glu Arg Ala Arg Val Gln Leu Gly Asn Val Val Thr Ala Ala
               20                  25                  30
   Ala Leu His Gly Gly Ile Arg Ile Ser Asp Gln Thr Thr Asn Ser Val
           35                  40                  45
   Glu Thr Val Val Gly Lys Gly Glu Ser Arg Val Leu Ile Gly Asn Glu
       50                  55                  60
   Tyr Gly Gly Lys Gly Phe Trp Asp Asn
   65                  70